# EUROPÄISCHE PATENTANMELDUNG

(11) **EP 1 985 565 A1**
(43) Veröffentlichungstag der Anmeldung: **29.10.2008**
(21) Anmeldenummer: 07008469.4
(22) Anmeldetag: 26.04.2007
(51) Int. Cl.: B65H 63/06, D01H 13/22, G01N 33/36, G01N 21/89

(54) **Vorrichtung zur optischen Fehlstellenermittlung in einem Garn oder Garnvorgänger**

(71) Anmelder: Gebrüder Loepfe AG, CH-8623 Wetzikon (CH)
(72) Erfinder: Mauron, Pascal, 8304 Wallisellen (CH); Hunziker, Christian, 8624 Grüt (CH); Maier, Joachim, 8280 Kreuzlingen (CH)
(74) Vertreter: Sutter, Kurt

(57) **Zusammenfassung**

Eine Vorrichtung zur Ermittlung von Fehlstellen in einem Garn (10) besitzt einen Messraum (14), in welchem die Garnqualität optisch gemessen wird. Seitlich am Messraum (14) ist ein Einführschlitz (15) vorgesehen, durch welchen das Garn (10) in den Messraum (14) eingeführt werden kann. Um das Eindringen von Umgebungslicht und/oder Verschmutzungen durch den Einführschlitz (15) zu verhindern, ist ein Verschlussorgan (16) vorgesehen. Das Verschlussorgan kann durch elastische Deformation oder von einem Antrieb (25) geöffnet werden.

## Beschreibung

Die Erfindung betrifft eine Vorrichtung zur optischen Fehlstellenermittlung in einem Garn oder Garnvorgänger.

Unter "Garnvorgänger" ist dabei eine Faseranordnung zu verstehen, wie sie im Verlauf der Garnherstellung auftritt, z.B. um ein Faservlies nach der Karde oder ein Faserbündel vor dem eigentlichen Spinnprozess.

Bei der Herstellung und Anwendung von Garnen muss eine dauernde Qualitätskontrolle durchgeführt werden. Hierzu sind verschiedene Verfahren bekannt, mit welchen die Qualität des Garns beziehungsweise des Garnvorgängers gemessen werden kann. Solche Verfahren messen beispielsweise die optische Transmission oder Reflektion eines Garns, um daraus Dünnstellen, Dickstellen, Nissen, Fremdfasern, Fehlfarben oder andere in optischer Weise erkennbare Defekte ermitteln zu können.

US 4 739 176 beschreibt eine Vorrichtung zur optischen Fehlstellenermittlung an einem Garn. Sie besitzt einen Messraum, an welchem ein oder zwei Lichtquellen sowie ein Lichtdetektor angeordnet sind. Die Lichtquellen beaufschlagen das Garn mit Messlicht, und der Detektor misst vom Garn zurückgeworfenes Licht. Am Messraum ist ein Einführschlitz vorgesehen, durch welchen das Garn von der Seite her in den Messraum eingeführt werden kann.

Es stellt sich die Aufgabe, die Messgenauigkeit eines solchen Geräts zu verbessern.

Diese Aufgabe wird von der Vorrichtung nach Anspruch 1 gelöst. Anspruchsgemäss ist demgemäss am Einführschlitz ein mindestens teilweise lichtundurchlässiges Verschlussorgan angeordnet. Durch dieses Verschlussorgan wird das Umgebungslicht besser vom Messraum ferngehalten, was eine genauere Messung erlaubt.

Vorzugsweise kann das Verschlussorgan mit einem Antrieb betätigt werden, oder es besitzt ein elastisches Verschlussteil, welches durch Einführen des Garns bzw. Garnvorgängers verformt werden kann. In diesem Fall ist die Vorrichtung auch besonders gut für Fälle geeignet, wo ein automatisches Einführen des Garns durch den Einführschlitz erforderlich ist, wie z.B. bei einem automatisierten Garnreiniger.

Die Erfindung ist besonders geeignet zur Anwendung in einem Garnreiniger. Sie kann jedoch auch an anderen Stellen eingesetzt werden, wo eine Überwachung des Garns bzw. Garnvorgängers notwendig ist.

Weitere vorteilhafte Ausführungen der Erfindung ergeben sich aus den abhängigen Ansprüchen sowie der nun folgenden Beschreibung anhand der Figuren. Dabei zeigen:
Figur 1 einen Schnitt durch eine erste Ausführung der erfindungsgemässen Vorrichtung,
Figur 2 einen Schnitt durch eine zweite Ausführung der erfindungsgemässen Vorrichtung,
Figur 3 einen Schnitt durch eine dritte Ausführung der erfindungsgemässen Vorrichtung und
Figur 4 einen Schnitt durch die Vorrichtung nach Figur 3 mit geöffnetem Einführschlitz.

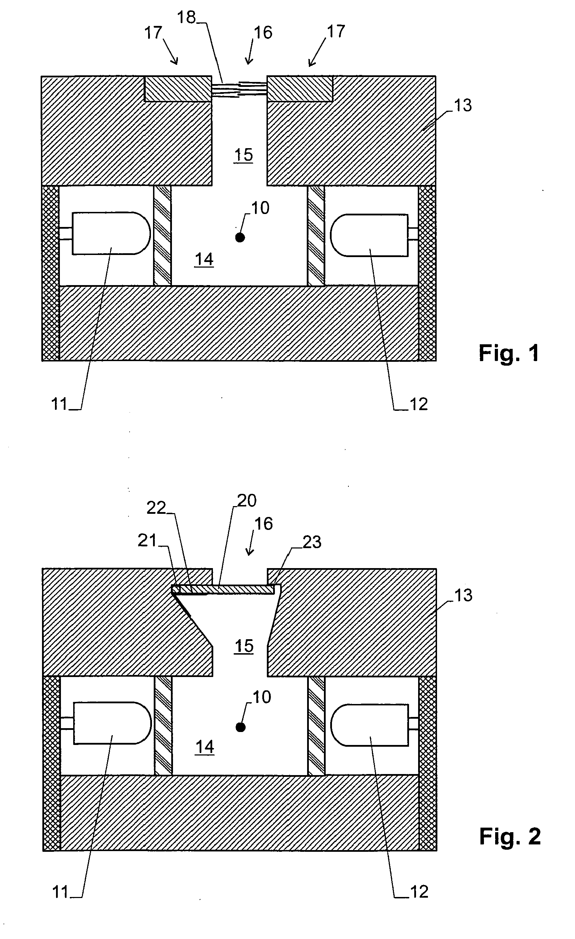

Ein erstes Beispiel einer erfindungsgemässen Vorrichtung wird in Figur 1 gezeigt. Dabei handelt es sich um eine Ausführung, bei welcher das Garn 10 zwischen einer Lichtquelle 11 und einem Lichtdetektor 12 angeordnet ist. Vorrichtungen mit dieser Anordnung sind dem Fachmann an sich bekannt. Sie dienen dazu, aufgrund des Schattenwurfs des Garns 10 die Dicke desselben zu messen. Es ist jedoch zu betonen, dass die Erfindung nicht auf Vorrichtungen dieser Art beschränkt ist. Sie kann für andere Arten von optischen oder teilweise optischen Messevorrichtungen angewendet werden, beispielsweise für Vorrichtungen, bei denen vom Garn zurückgeworfenes Licht detektiert wird. Auch kann die Zahl der Lichtquellen und Lichtsensoren variieren. Beispielsweise sind dem Fachmann auch Anordnungen mit zwei oder mehr Lichtquellen und/oder Lichtdetektoren bekannt.

Die Vorrichtung nach Figur 1 besitzt ein lichtundurchlässiges Gehäuse 13, welches einen Messraum 14 bildet, in welchem das zu messende Garn 10 läuft. Der Messraum 14 ist ein länglicher Raum, welcher sich senkrecht zur Zeichenebene von Figur 1 erstreckt und an seinen Enden mindestens teilweise bis ganz zu sein kann. Seitlich am Messraum 14 ist ein Einführschlitz 15 angeordnet, der sich über die ganze Länge des Messraums 14 erstreckt. Der Einführschlitz 15 dient dazu, das Garn 10 seitlich in den Messraum 14 einzuführen.

Am Einführschlitz 15 ist ein Verschlussorgan 16 angebracht. Dieses dient dazu, den Eintritt von Umgebungslicht und Verschmutzungen in den Messraum 14 zu erschweren oder zu verhindern.

In der Ausführung nach Figur 1 wird das Verschlussorgan 16 von zwei Bürsten 17 gebildet, deren Haare 18 von gegenüberliegenden Seiten in den Einführschlitz 15 ragen und einander überlappen. Die Haare 18 bilden ein elastisches Verschlussteil, welches beim Einführen des Garns in den Einführschlitz 15 verformt wird und so den Weg für das Garn 10 freigibt.

Die Bürsten 17 erstrecken sich in Richtung senkrecht zur Zeichenebene vorzugsweise im Wesentlichen über die ganze Länge des Schlitzes 15, so dass der Messraum 14 möglichst gut geschützt ist.

Eine zweite Ausführung der Erfindung ist in Figur 2 dargestellt. Die dort gezeigte Vorrichtung ist an sich gleich aufgebaut wie jene nach Figur 1, mit der Ausnahme, dass das Verschlussorgan 16 von einer Verschlussklappe 20 gebildet wird. Die Verschlussklappe 20 kann um ein Scharnier 21 verschwenkt werden, dessen Schwenkachse parallel zum Einführschlitz 15 verläuft. Eine Scharnierfeder 22 hält die Verschlussklappe 20 normalerweise in geschlossener Stellung. In dieser geschlossenen Stellung erstreckt sich die Verschlussklappe 20 über den Einführschlitz 15 und verschliesst diesen. Auf der dem Scharnier 21 gegenüberliegenden Seite des Einführschlitzes 15 liegt die Verschlussklappe 20 mit der Kraft der Scharnierfeder 22 an einer Nase 23 auf. Wird das Garn 10 in den Einführschlitz 15 eingeführt, so wird die Verschlussklappe 20 gegen die Kraft des Garns 10 um das Scharnier 21 geschwenkt und öffnet sich. Nach dem Einführen des Garns 10 schliesst sich die Verschlussklappe 20 dank der Feder 22 automatisch.

Bei der Ausführung nach Figur 2 kann das Garn 10 zwar in den Einführschlitz 15 eingeführt werden, eine Entfernung des Garns 10 durch den Einführschlitz 15 ist jedoch, im Gegensatz zur Ausführung nach Figur 1, nicht möglich.

Bei den soweit gezeigten Ausführungen der Erfindung handelt es sich um Lösungen, bei welchen das Verschlussorgan 16 durch die Kraft des Garns betätigt wird.

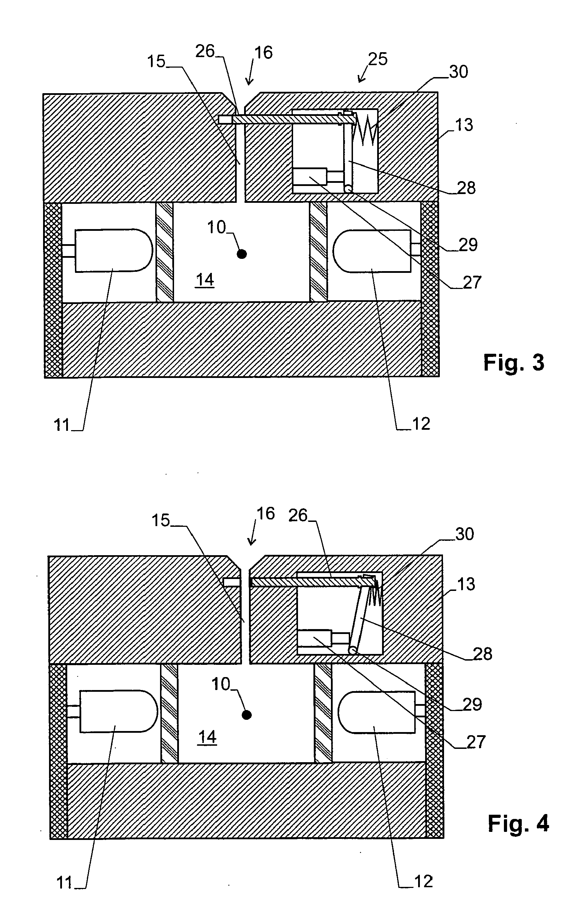

Figur 3 zeigt eine Lösung, bei welcher das Verschlussorgan 16 von einem Antrieb 25 aktiv bewegt wird. In der dargestellten Ausführung ist ein Verschlussschieber 26 vorgesehen, welcher in den Einführschlitz 15 hinein geschoben werden kann. Der Antrieb 25 besteht aus einem piezoelektrischen Stellglied 27, dessen Länge durch eine angelegte elektrische Spannung geändert werden kann, und das einen Schwenkhebel 28 betätigt. An einem Ende ist der Schwenkhebel 28 an einem Schwenklager 29 angelenkt, und am anderen Ende ist er mit dem Verschlussschieber 26 so gekoppelt, dass er diesen zu ziehen und zu stossen vermag. Zudem ist eine Feder 30 vorgesehen, welche den Schwenkhebel 28 gegen das Stellglied 27 und den Verschlussschieber 26 in den Einführschlitz 15 drückt.

Wird am Stellglied 27 eine Spannung angelegt, so dehnt sich dieses aus, der Schwenkhebel 28 wird verschwenkt, und der Verschlussschieber 26 wird aus dem Einführschlitz 15 gezogen und gibt diesen frei. Die entsprechende Situation ist in Figur 4 dargestellt.

Da das Stellglied 27 relativ nahe am Schwenklager 29 des Schwenkhebels 28 angreift, kann dessen relativ geringe Hubbewegung in eine ausreichend lange Schwenkbewegung am freien Ende des Schwenkhebels 28 umgesetzt werden.

Anstelle eines piezoelektrischen Stellglieds 27 kann z.B. auch ein elektromagnetischer Antrieb vorgesehen sein. Das Verschlussorgan kann anstelle eines Verschlussschiebers 26 z.B. auch eine von einem Antrieb betätigbare Schwenkklappe aufweisen.

Besonders geeignet ist die vorliegende Erfindung für die Verwendung in Garnreinigern, bei welchen fehlerhafte Garnstellen automatisch herausgeschnitten werden. Derartige Garnreiniger müssen das Garn 10 nach dem Herausschneiden der Garnstelle wieder in den Messraum 14 einführen, was bei den beschriebenen Ausführungen ohne weiteres möglich ist. Erfolgt die Einführung des Garns 10 immer manuell, kann auch ein manuell betätigtbares Verschlussorgan, wie z.B. eine von Hand öffenbare Klappe, vorgesehen sein.

Auf jeden Fall sollte das Verschlussorgan 16 zumindest teilweise lichtundurchlässig sein, so dass es Umgebungslicht vom Messraum 14 fernzuhalten vermag.

Wie eingangs erwähnt, kann die Vorrichtung nicht nur für Garne sondern auch für Garnvorgänger, zum Beispiel in Form eines Faserbündels nach der Karde in einer Spinnereianlage, eingesetzt werden.

## Patentansprüche

1. Vorrichtung zur optischen Fehlstellenermittlung in einem Garn oder Garnvorgänger
mit einem Messraum (14), an welchem mindestens eine Lichtquelle (11) zum Beaufschlagen des Garns bzw. Garnvorgängers mit Messlicht und mindestens ein Lichtdetektor (12) zum Messen von Messlicht, welches vom Garn bzw. Garnvorgänger beeinflusst worden ist, angeordnet ist und
mit einem Einführschlitz (15), durch welchen das Garn in den Messraum (14) einführbar ist,
**dadurch gekennzeichnet, dass** am Einführschlitz (15) ein mindestens teilweise lichtundurchlässiges Verschlussorgan (16) angeordnet ist.

2. Vorrichtung nach Anspruch 1, wobei das Verschlussorgan (16) beweglich ist.

3. Vorrichtung nach einem der vorangehenden Ansprüche, wobei das Verschlussorgan (16) von einem Antrieb (25) betätigbar ist.

4. Vorrichtung nach Anspruch 3, wobei der Antrieb (25) piezoelektrisch ist.

5. Vorrichtung nach einem der vorangehenden Ansprüche, wobei das Verschlussorgan (16) mindestens eine schwenkbare Verschlussklappe (20) aufweist.

6. Vorrichtung nach einem der vorangehenden Ansprüche, wobei das Verschlussorgan (16) mindestens einen verschiebbaren Verschlussschieber (26) aufweist.

7. Vorrichtung nach einem der vorangehenden Ansprüche, wobei das Verschlussorgan (16) mindestens ein elastisches Verschlussteil (18, 22) aufweist, welches durch Einführen des Garns bzw. Garnvorgängers zum Öffnen des Einführschlitzes (15) verformbar ist.

8. Vorrichtung nach Anspruch 7, wobei das Verschlussorgan (16) eine Vielzahl elastischer Haare (18) aufweist, welche in den Einführschlitz ragen.

9. Garnreiniger mit einer Vorrichtung nach einem der vorangehenden Ansprüche.
